# EUROPÄISCHE PATENTSCHRIFT

(11) **EP 1 909 568 B1**
(45) Veröffentlichungstag und Bekanntmachung des Hinweises auf die Patenterteilung: **10.12.2008**
(21) Anmeldenummer: 06777733.4
(22) Anmeldetag: 12.07.2006
(51) Int. Cl.: A01N 31/02, A01N 35/02, A01N 31/08, A01N 31/14

(54) **STOFFGEMISCH ZUR BEHANDLUNG VON WIRBELLOSEN UND WIRBELTIEREN BEI TIERISCHEM SCHÄDLINGSBEFALL**
MIXTURE OF SUBSTANCES FOR TREATING INVERTEBRATE AND VERTEBRATE PARASITES THAT ATTACK ANIMALS
MELANGE DE SUBSTANCES POUR LE TRAITEMENT DES PARASITES VERTEBRES ET INVERTEBRES DES ANIMAUX

(30) Priorität: 12.07.2005 DE 102005032918
(43) Veröffentlichungstag der Anmeldung: 16.04.2008
(73) Patentinhaber: Schneider, Frank, 67067 Ludwigshafen (DE); Schütz, Ralf, 68169 Mannheim (DE)
(72) Erfinder: SCHÜTZ, Ralf, 68169 Mannheim (DE)
(74) Vertreter: Thews, Karl
(86) Internationale Anmeldenummer: PCT/EP2006/064164
(87) Internationale Veröffentlichungsnummer: WO 2007/023028

(56) Entgegenhaltungen:
- EP-A- 0 252 278
- WO-A-01/00034
- WO-A-01/95726
- WO-A-97/25404
- DE-A1- 19 917 948
- US-A1- 2003 194 454
- DATABASE WPI Week 198248 Derwent Publications Ltd., London, GB; AN 1982-03407J XP002436330 & JP 57 171906 A (KIGOSHI Y) 22. Oktober 1982 (1982-10-22)

## Beschreibung

Die Erfindung bezieht sich auf ein Gemisch, insbesondere ein Bio- oder Insektizid zur äußerlichen und innerlichen therapeutischen Behandlung von wirbellosen Tieren und Wirbeltieren, insbesondere zur Behandlung von Insekten und Gliedertieren bei tierischem Schädlingsbefall. Zudem bezieht sich die Erfindung auf die Verwendung eines solchen Gemisches zur Herstellung eines Medikaments für die entsprechende Behandlung von Insekten. Schädlinge sind in diesem Zusammenhang insbesondere der Gattung der wirbellosen Tiere zuzuordnen.

Es sind bereits zahlreiche Gemische oder Biozide zur Bekämpfung von Schädlingen wie bspw. die Varroamilbe, die auch Honigbienen und deren Larven befällt, oder die Milbe Acarapis woodi (USA) bekannt. Diese Stoffe sind in mehreren Staaten teilweise nicht zugelassen und teilweise verschreibungspflichtig.

Gerade bei der Behandlung von Nutzinsekten wie Honigbienen kommt es darauf an, dass einerseits möglichst wenig Bienen durch die Behandlung sterben und andererseits das entsprechende Biozid weitestgehend rückstandsfrei eingesetzt werden kann, d. h. dass keine Rückstände im Bienenprodukt wie beispielsweise im Honig oder im Wachs verbleiben. Für den Fall, dass Rückstände aufgrund der Konzentration oder der Applikation des Biozids unumgänglich sind, sollte das Biozid für den Menschen gesundheitlich unbedenklich sein.

Ameisensäure und Milchsäure sind einige der wenigen Vertreter der bekannten Bio- oder Insektizide, die rückstandsfrei oder zumindest ohne gesundheitliche Nachteile für den Menschen eingesetzt werden können. Die Sterblichkeitsrate der Nutzinsekten ist aber auch hier problematisch.

Die unter dem Markennamen "Apiguar^{®}" mit dem Wirkstoff Thymol und unter dem Markennamen "Bayvarol^{®}" mit dem Wirkstoff Flumethrin sowie unter dem Markennamen "Perizin^{®}" mit dem Wirkstoff Coumaphos erhältlichen Biozide gehören zu der Gruppe der Biozide, die hinsichtlich der verbleibenden Rückstände in den Honigprodukten teilweise als gesundheitlich problematisch für den Menschen eingestuft werden.

Des Weiteren sind Biozide mit den Markennamen "Apistan^{®}", "Apitol^{®}", "Apivar^{®}", "Supona^{®}", "Birlane^{®}", "Asuntol^{®}", "TakTic^{®}" und "Bumentran^{®}" bekannt, die aufgrund der für den Menschen gesundheitlich bedenklichen Rückstände in den Honigprodukten und im Wachs auch in der Bundesrepublik Deutschland nicht zugelassen sind.

Zahlreiche Versuche, die Milbe, insbesondere die Varroamilbe oder Spulwürmer (Nematoden), unschädlich zu machen oder langfristig einzudämmen und dabei keine für den Menschen gesundheitlich bedenklichen Stoffe einzusetzen, sind bisher mehr oder weniger erfolglos geblieben. Im Jahr 2003 ist durch die Varroamilbe, die Bienenvölker befallen hat, allein in den USA ein Schaden in Höhe von 10 Mrd. US$ entstanden.

Selbst hochwirksame Bio- oder Insektizide verlieren, wie festgestellt wurde, aufgrund von Resistenzbildung seitens des Schädlings in den letzten Jahren zunehmend an Wirksamkeit.

Die Bekämpfung der Varroatose mit diesen herkömmlichen, kommerziellen Akariziden führt demnach zu keinem zufrieden stellenden Ergebnis. Es ist als Alternative bekannt, verschiedene etherische Öle, also flüchtige natürliche oder naturidentische sowie synthetische Duftstoffe auf Basis einer aromatischen Hydroxyverbindung in verschiedenen Konzentrationen zur Bekämpfung von Schädlingen einzusetzen.

Problematisch hierbei ist die Art der Applikation und die Steuerung der Dosis, der zu oft auch die Nutzinsekten zum Opfer fallen. Bei den etherischen Ölen sowie bei den chemischen Reinstoffen stellt sich das Problem der Löslichkeit in Wasser. Die Basis der aromatischen Hydroxyverbindungen bilden meist Phenole oder Phenolester, die ebenso wie Phenol selbst nahezu wasserunlöslich sind.

Im Laborversuch kann die Steuerung der Dosis durch entsprechend aufwendige Versuchsanordnungen gewährleistet werden, jedoch ist für die praktische Anwendung im Feld wie bspw. bei Bienenstöcken bisher keine zufrieden stellende Lösung bekannt, die Dosis ausreichend hoch, aber unterhalb der Schadensgrenze einzustellen.

Reine etherische Öle wurden bei Feldversuchen teilweise durch Verdunsten in der Umgebungsluft oder teilweise durch direktes Auftragen auf die Schädlinge appliziert. Eine wiederholbare Konzentration, die ausreichend und schnell wirksam ist und keinen zu großen Schaden an den Nutzinsekten anrichtet, konnte durch diese Methode bisher nicht erreicht werden.

Aus der DE 600 14 100 T2 ist gemäß Abs. 0027 bekannt, als Träger für Pestizidzusammensetzungen Ketone wie bspw. Aceton oder Alkohole einzusetzen. Es werden zwei Formulierungsbeispiele unter Verwendung von Aceton beschrieben. Im Formulierungsbeispiel gemäß Abs. 0041 wird Phenethylproprionat in Aceton aufgelöst und mit Rosmarinöl, Pfefferminzöl und Eugenol vermischt. Im Formulierungsbeispiel gemäß Abs. 0043 wird Methylsalicylat in Aceton aufgelöst und mit Pfefferminzöl, Eugenol und Phenethylproprionat vermischt.

Aus der US 2003/0194454 ist zur Bekämpfung von Schädlingen gemäß S. 18 ff. der Einsatz von 5 % bis zu 75 % eines oder mehrerer etherischen Öle unter Verwendung eines geeigneten Trägers beschrieben. Konzentrationen von durchschnittlich 35 % sind bei dieser Methode notwendig, damit eine ausreichend hohe Mortalitätsrate der Schädlinge erreicht werden kann. Allerdings ist bei dieser Methode die Gefahr groß, dass das etherische Öl in zu hohen Konzentrationen das Nutzinsekt schädigt, d. h. es sterben nicht nur die Schädlinge, sondern auch der Patient, in den beschriebenen Fällen also die Bienen. Bei einer zu hohen Dosis von bspw. Nelkenöl in einem Bienenstock sterben mehr als 90 % der Bienen dieses Stockes.

Aufgabe ist es, ein Gemisch und somit auch eine Applikationsform bereitzustellen, das eine problemlose Nutzung eines etherischen Öls ermöglicht, d. h. sich nicht negativ auf das Wohlbefinden des Nutzinsekts auswirkt und eine Mortalitätsrate des Schädlings von mindestens 95 % herbeiführt, und das im Falle der Behandlung von Bienen gleichzeitig nahezu ohne Rückstände im Honig, sodass das Gemisch für die Gesundheit des Menschen bedenkenlos einsetzbar ist.

Es wurde durch Zufall gefunden, dass ein Gemisch bestehend aus einem primären und/oder sekundären Alkohol als Komponente A, einem Keton als Komponente B und einem Stoff eines etherischen Öls als Komponente C dann eine gemessen an der Mortalitätsrate des Schädlings im Vergleich mit einer Mischung aus Wasser, etherischem Öl und Aceton um einen Faktor 300 bessere Wirkung entfaltet, wenn dem Stoffgemisch als weitere Komponente D ein Oxidationsmittel wie bspw. Wasserstoffperoxid (H₂O₂) zugesetzt wird. Das Oxidationsmittel wird bevorzugt mit den Komponenten A und B gemischt, wodurch eine Redox-Reaktion in Gang gesetzt wird, durch die das Oxidationsmittel reduziert und zumindest eine der Komponenten A und B teilweise oxidiert wird. Unmittelbar danach oder nach Zumischen weiterer Komponenten wird die Komponente C dem Gemisch mit der Komponente D beigemischt. Diese durch die Oxidation der Komponenten A und/oder B entstehende sogenannte Oxidkomponente OX bewirkt eine Verbesserung, durch die weitestgehend alle Schädlinge abgetötet werden, ohne das Nutzinsekt zu schädigen.

Aufgrund der Zusammenwirkung dieser Stoffe wird ein synergistischer Effekt erzielt, da gleichzeitig mit der Reduzierung der Konzentration des etherischen Öls von durchschnittlich 35 % auf weniger als 5 Gew.% eine Erhöhung der Überlebensrate der Insekten und eine Erhöhung der Mortalitätsrate der Schädlinge erreicht wird.

Der Zufall war durch einen Abfüllfehler bedingt, bei dem ein Gemisch aus verschiedenen Alkoholen und Aceton in einen Behälter mit einer Restmenge Wasserstoffperoxid gemischt und nachträglich ein etherisches Öl zugemischt wurde. Bei der Mischung mit Wasserstoffperoxid wurde festgestellt, dass in dem zunächst klaren Gemisch eine geringe Menge eines Stoffes in Form weißer Flocken ausfällt. Der Neugierde des Erfinders ist es zuzurechnen, dass das zunächst als unbrauchbar angesehene Gemisch an einem Bienenvolk ausprobiert wurde, das ohnehin aufgrund eines starken Milbenbefalls in Quarantäne war und hätte vernichtet werden müssen.

Das Bienenvolk wurde durch dieses zufällige Gemisch vollständig vom Milbenbefall befreit.

Zum Anmeldezeitpunkt war die genaue Wirkungsweise noch nicht bekannt. Es wird angenommen, dass durch die Zugabe des Oxidationsmittels Wasserstoffperoxid eine Redox-Reaktion mit einem der Alkohole oder mit Aceton erfolgte. Die aus dieser Reaktion entstehenden Stoffe, insbesondere die Oxidkomponente OX, war für den Anmelder zum Anmeldezeitpunkt nicht definierbar.

Insgesamt 24 Vergleichsversuche, von denen ein repräsentatives Ergebnis anhand nachstehender Tabelle erläutert wird, zeigen diesen synergistischen Effekt. Dabei wurden jeweils zwei Bienenvölker mit dem erfindungsgemäßen Mittel namens NOE SS 25 und zwei Völker mit einer Mischung aus 33 % Wasser, 54 % Alkohol, 3 % Aceton und 10 % Thymol 14 Wochen lang behandelt. Die Besatzdichte betrug anfänglich etwa 25.000 Bienen je Volk. Die Bienenvölker waren in sogenannten Herolds- oder Zeitlerbeuten mit je 2 Beuten à 10 Zargen untergebracht. Die Zahlenwerte in der Tabelle stellen den Milbenfall dar, d. h. die Fallquote der toten Milben, die im Bienenstock herunterfallen. Die Genauigkeit der Quote wurde durch wöchentliches Wechseln des Bodenbelags im Bienenstock gewährleistet.

| Mittel | NOE SS 23 | | Thymol 10 | |
|---|---|---|---|---|
| Völker | BV 1 | BV 2 | BV 3 | BV 4 |
| * 1. | 0 | 0 | 0 | 0 |
| * 2. | 0 | 0 | 0 | 0 |
| * 3. | 0 | 2 | 5 | 3 |
| * 4. | 0 | 0 | 8 | 9 |
| 5. | 0 | 1 | 9 | 14 |
| 6. | 0 | 0 | 15 | 17 |
| 7. | 2 | 4 | 33 | 23 |
| * 8. | 0 | 2 | 35 | 37 |
| * 9. | 1 | 1 | 42 | 39 |
| * 10. | 1 | 0 | 47 | 58 |
| ** 11 | 1 | 2 | 750 | 1050 |
| ** 12. | 4 | 7 | 1050 | 1350 |
| 13. | 6 | 3 | 19 | 14 |
| 14. | 1 | 1 | 3 | 2 |
| total | 16 | 23 | 2016 | 2616 |

In den ersten vier Wochen sowie in der 8. bis 10. Woche wurde einmal wöchentlich das jeweilige Stoffgemisch in einer Menge von 70 ml durch Sprühverfahren in die Zargen eingebracht. In der Woche 11 und 12 wurden alle 4 Völker zur Kontrolle des noch vorhandenen Milbenbesatzes mit Perizin^{®} nach Vorschrift im Träufelverfahren behandelt.

Der Milbenfall bei BV 1 und BV 2 war bis zur Behandlung mit Perizin^{®} konstant niedrig und lag im Wochenschnitt bei 0,7 Milben. Der Milbenfall bei BV 3 und BV 4 war stetig steigend und betrug im Wochenschnitt 19,7 Milben, also annähernd Faktor 30. Nach 10 Wochen wurde zur Erfassung der noch im Stock lebenden Milben eine Behandlung mit Perizin^{®} durchgeführt. Bei BV 1 und BV 2 wurden über zwei Wochen im Mittel 7 Milben gezählt. Bei BV 2 und BV 3 waren es im Mittel 2.100 Milben, also 300 Mal mehr.

Dieses Ergebnis zeigt, dass das erfindungsgemäße Gemisch nicht nur die Vermehrung der Milben erfolgreich verhindert, sondern auch alle Milben bekämpft. Das Thymolgemisch verhindert die Vermehrung nicht und erfasst nur einen Bruchteil der Milben.

Hinzu kommt, dass die Bienensterblichkeit bei NOE SS 23 wesentlich geringer war als bei Thymol 10. Die Bienensterblichkeit bei N betrug ca. 40 pro Woche, was knapp über der natürlichen Rate von ca. 30 Bienen liegt. trug die Bienensterblichkeit ca. 270 pro Woche, also mehr als 15 % aller Bienen im Stock über den Behandlungszeitraum von 14 Wochen.

Im Vorfeld der Vergleichsversuche wurde das erfindungsgemäße Gemisch an einem ersten Bienenvolk angewendet und mit einem unbehandelten Bienenvolk verglichen. Diese Versuche werden im Anschluss an die Beschreibung erläutert.

Der Vorteil, dass der Schädling bei der Applikation des erfindungsgemäßen Gemisches eingeht und gleichzeitig das Nutzinsekt keinen Schaden nimmt, ist darauf zurückzuführen, dass die Oxidkomponente OX einerseits eine bessere Dosierung des etherischen Öls ermöglicht und andererseits aufgrund der Dosierung tödlich auf den Schädling wirkt. Von der Dosierung bzw. von der Konzentration der Komponenten hängt es nämlich letztendlich ab, auf welche Tiere das Gemisch tödlich wirkt. Bei kleinen Tieren ist eine weniger starke Konzentration ausreichend, durch die größere Tiere nicht zu Schaden kommen. Dieser Effekt wird auch bei der Bekämpfung von Parasiten wie bspw. Milben angewendet, da die Milben um ein Vielfaches kleiner sind als bspw. Bienen.

Insbesondere von Vorteil ist dabei, dass sich die wasserlösliche Eigenschaft der Oxidkomponente OX derart verändert, dass das etherische Öl oder das Phenol wesentlich besser gelöst werden kann und damit eine homogene Dosierung möglich wird. Entgegen der bisherigen Methode werden nicht die wasserunlöslichen Moleküle des etherischen Öls durch Aceton gelöst, sondern durch die Oxidkomponente OX. Wasser dient wie nachstehend beschrieben zur Verdünnung des erfindungsgemäßen Gemisches.

Der Alkohol dient als unschädliche Trägersubstanz, mit der der Schädling und das zu behandelnde Tier aufgrund der niedrigen Oberflächenspannung benetzt wird. Die Dosis der Oxidkomponente OX ist durch diese Möglichkeit der homogenen Verteilung exakt einstellbar und aufgrund des reduzierten Konzentrationsbereiches von weniger als 5 Gew.% etherischen Öls für das Nutzinsekt nicht schädlich, aber für die Bekämpfung des Schädlings ausreichend.

Der Alkohol als Mittel mit einer sehr guten Diffusions- und Benetzungseigenschaft und einer sehr vorteilhaften Eigenschaft als Lösungsmittel hat die Wirkung, dass nach der Benetzung der Alkohol zusammen mit dem Keton und dem etherischen Öl bzw. der Oxidkomponente OX durch die Zellwände des Nestes der Milbe und auch durch die Haut oder Cuticulla des Schädlings hindurchdringt. Ein Beispiel für eine Zellwand wäre die Verdeckelung der Bienenlarve, die als Nest für die Milbe dient. Alkohol ist zudem für das zu behandelnde Tier weitestgehend unschädlich und wirkt betäubend.

Ferner hat dieses Gemisch die vorteilhafte Eigenschaft, dass sowohl das zu behandelnde Tier als auch der Schädling vollständig benetzt werden und das Gemisch aufgrund seiner äußerst niedrigen Oberflächenspannung auch durch Körperöffnungen des zu behandelnden Tiers und des Schädlings eindringt, ohne dass es der Schädling vermeiden kann. Das Gemisch dringt bspw. bei Spinnen durch die Körperöffnung zur Nahrungsaufnahme in die. Spinne ein, wodurch die Schädlinge im Körperinneren, im Verdauungstrakt der Spinne ebenfalls benetzt werden und das Gemisch dort auch entsprechend in den Schädling eindringt und diesen absterben lässt.

Das Gemisch wird entweder durch Verdunstung oder in Form von feinen Tröpfchen durch Versprühen in das Nest des zu behandelnden Tiers wie bspw. in den Bienenstock appliziert. Eine weitere Applikationsform besteht durch Aufbringen von Sprühnebel oder Tropfen auf dem einzelnen zu behandelnden Tier selbst.

Das Gemisch enthält im Speziellen eine Komponente A aus mindestens einem primären oder sekundären Alkohol der allgemeinen Formel R1-OH, wobei R1 ein an die Hydroxylgruppe anschließender verzweigter oder unverzweigter Alkylrest mit 1 bis 12, bevorzugt mit 1 bis 6 Kohlenstoffatomen ist, eine Komponente B der Familie der Ketone bestehend aus mindestens einer Carbonylverbindung der allgemeinen Formel R2-CO-R2', wobei R2 oder R2' ein an die Carbonylgruppe anschließender verzweigter oder unverzweigter Aryl- oder Alkylrest oder Wasserstoff ist. R2 und R2' können gleich oder verschieden sein. Die Komponente C enthält mindestens einen Stoff auf Basis der aromatischen Hydroxyverbindung Benzen der allgemeinen Formel C₆H₆ aus der Familie der etherischen Öle.

Hergestellt wird das Gemisch durch das Mischen von wenigstens einer der Komponenten A, B und C mit wenigstens einer weiteren Komponente D und das anschließende Mischen aller beteiligten Komponenten, wobei die Komponente D ein Oxidationsmittel ist, das mit wenigstens einer der Komponenten A bis C derart redox reagiert, dass das Oxidationsmittel reduziert wird. Bevorzugt werden die Komponenten A und B mit der Komponente D gemischt und anschließend die Komponente C hinzugemischt.

Ein etherisches Öl im Sinne der Erfindung ist ein Gemisch, das größtenteils aus einer aromatischen Hydroxyverbindung der Form R3-OH besteht. R3 besteht aus mindestens einem ringförmigen Arylrest, der seinerseits ein- oder mehrfach substituiert ist. An den Arylrest schließt mindestens eine organische Verbindung R3' an, wobei R3' ein Aryl-, Alkyl- oder Cycloalkylrest ist. Bevorzugt werden ringförmige Arylreste mit 6 Kohlenstoffatomen. Der Alkylrest ist dabei verzweigt oder unverzweigt.

Synthetische und nahezu reine Wirkstoffe der etherischen Öle liegen aber dennoch in der Praxis im Gemisch mit verschiedenen anderen Stoffen und Verunreinigungen vor. In natürlichen etherischen Ölen sind meist nur bis zu 70 % der chemisch reinen aromatischen Hydroxyverbindung enthalten, deshalb können neben der im Vordergrund stehenden aromatischen Hydroxyverbindung Hunderte von weiteren Stoffen und Verunreinigungen enthalten sein.

Besonders gute Ergebnisse wurden dadurch erreicht, dass die Komponente A zumindest 1-Propanol mit der Summenformel CH₃-CH₂-CH₂-OH und/oder 2-Propanol mit der Summenformel CH₃-CH-OH-CH₃ enthält. Propanol hat gegenüber anderen Alkoholen mit längeren Ketten die vorteilhafte Eigenschaft, dass er weniger gesundheitsschädlich ist, leicht flüchtig ist und für einen schnellen Transport des Gemischs sorgt. Grundsätzlich hat Propanol den Vorteil, dass er sich mit den übrigen Zusätzen sehr gut mischt und kostengünstig hergestellt werden kann.

Es ist weiterhin vorteilhaft, dass die Komponente B zumindest Aceton (Propanon) mit der Summenformel CH₃-CO-CH₃ enthält, wodurch die Wirksamkeit weiter gesteigert wurde. Aceton hat gegenüber anderen Ketonen die vorteilhafte Eigenschaft, dass es sehr gute Eigenschaften als Lösungsvermittler hat. Die einzelnen Komponenten des Gemischs wie bspw. die Komponenten A und C weisen unterschiedliche Dichten auf, was auf molekularer Ebene zu Ansammlungen einzelner Komponenten führt. Solche Ansammlungen bzw. ungleiche Mischungsverhältnisse müssen unbedingt vermieden werden, da durch zu hohe bzw. zu niedrige Konzentrationen der einzelnen Komponenten innerhalb der jeweiligen Dosiermenge die Wirkung des gesamten Gemisches verloren geht oder das Nutzinsekt Schaden nehmen kann. Eine dauerhafte Gleichverteilung im Gemisch von Wasser, Alkohol und Öl wird auch durch die Oxidation der aromatischen Verbindung im Öl erreicht.

Ferner ist es vorteilhaft, dass die Komponente C wenigstens Phenol der allgemeinen Formel C₆H₅-OH und/oder ein Phenolether der allgemeinen Formel C₆H₅-O-R enthält. Phenole und Phenolether sind bspw. im Nelkenöl (Eugenol) enthalten, mit dem besonders gute Ergebnisse erzielt werden.

Alkohol hat den vorteilhaften Effekt, dass die Körperöffnung des Insektes wie bspw. die einer Spinne oder einer Biene sowie die Körperöffnung des Schädlings zur Aufnahme von Nahrung vollständig mit dem Gemisch benetzt wird. Das Gemisch dringt nach dem Benetzen in die Körperöffnung ein, ohne dass sich der Patient oder der Schädling dagegen wehren kann. Der Benetzungsvorgang pflanzt sich also in vorteilhafter Weise oral ins Körperinnere fort, sodass das Gemisch bis in den gesamten Verdauungstrakt vordringt, ohne dass der Patient oder der Schädling durch Verschließen der Körperöffnung dieses verhindern könnte.

Im Falle der Behandlung von Spinnen, die mit Spulwürmern (Nematoden) befallen sind, dringt das Gemisch von selbst bis in den Verdauungstrakt der Spinne vor, nachdem diese im Bereich der Körperöffnung mit dem Gemisch beträufelt wurde. Der Spulwurm selbst wird ebenfalls durch das Gemisch benetzt, das auch in ihn eindringt und seine Wirkung entfaltet.

Eine weitere Verbesserung der Ergebnisse wird dadurch erreicht, dass als Komponente C als etherisches Öl Nelkenöl oder die reine Komponente Eugenol verwendet wird. Nelkenöl oder Eugenol sind dafür bekannt, dass diese bereits bei sehr geringer Dosis recht wirksam sind, es allerdings schon bei einer geringen Überschreitung der Dosis zu einer massiven Schädigung der zu behandelnden Tiere kommt. Die Fachleute raten von der Verwendung dieser beiden kritischen Öle ab. Erst die erfindungsgemäße Idee, eine Gleichverteilung des etherischen Öls im Alkohol als Trägersubstanz mit Hilfe von Oxidation zu erreichen und somit die Dosis mit Hilfe der leicht regelbaren Verdunstungsrate von Alkohol und Wasser einzustellen und somit geringe, aber ausreichend effiziente Konzentrationen dieser kritischen Öle einsetzen zu können, macht den Einsatz von Nelkenöl oder Eugenol sicher.

Neben Nelkenöl oder Eugenol kommen auch weitere etherische Öle oder deren reine Komponenten (Reinsubstanzen) in Kombination mit einem oder mehreren Ölen bzw. Komponenten oder allein in vorteilhafter Weise zum Einsatz. Vertreter weiterer hinsichtlich der Schädigung des zu behandelnden Tieres kritischer Öle sind Knoblauchöl, Oreganoöl, Thymianöl, Wermutöl und Zwiebelöl. Weniger kritische Öle sind Allysenföl, Anisöl, Anonaöl, Baldrianöl, Bergamotöl, Bohnenkrautöl, Dillöl, Essigbaumöl, Eukalyptusöl, Fenchelöl, Föhreöl, Galbanumöl, Gänsefußöl, Geraniumöl, Gewürznelkenöl, Grapefruitöl, Grüne-Minze-Öl, Kamillenöl, Kardamomöl, Kiefernöl, Korianderöl, Kümmelöl, Latschenkiefernöl, Lavendelöl, Lilienöl, Lorbeeröl, Margeritenöl, Majoranöl, Mandarinöl, Melissenöl, Minzöl, Muskatnussöl, Niembaumöl, Olivenöl, Orangenblütenöl, Pfefferminzöl, Rosenöl, Rosmarinöl, Salbeiöl, Sandelholzöl, Sassafrasöl, Sellerieöl, Sonnenblumenöl, Spiköl, Tannennadelöl, Vanillin, Veilchenöl, Wacholderöl, Weinhefeöl, Wintergrünöl, Ysopöl, Zederöl, Zimtöl, Zitronenöl, Zitrusöl.

Weiter vorteilhaft sind chemisch reine Komponenten von etherischen Ölen, die wiederum in Kombination mit anderen reinen Komponenten oder mit anderen Ölen oder allein zum Einsatz kommen. Chemisch reine Komponenten im Sinne der Erfindung sind bspw. Acetyleugenol, Anethol, a-Pinen, a-Terpinen, a-Thujon, Benzoesäure, Benzylalkohol, Bornylacetat, Camphen, Caryophyllen, Citral, Citronella, Citronellol, Coumarin, Decanal, Elemol, Eucalyptol, Eugenol, Eukalyptol, Geraniol, Isoeugenol, Isoeugenol, Kampfer, Limonen, Linalool, Linalylacetat, Menthol, Menthon, Nerolido, Octenal, p-Cymol, Phenylacetylen, Pinen, Safrol, Terpineol, Thymol, y-Terpinen, Zimtaldehyd.

Die einzelnen Öle oder Komponenten haben zusätzlich unterschiedliche narkotisierende und stimulierende Wirkung, sodass eine Mischung mehrerer dieser Stoffe je nach Art des Schädlings und abhängig von der Art des zu behandelnden Tiers mindestens genauso gute, wenn nicht sogar bessere Eigenschaften hat als Nelkenöl.

Besonders vorteilhaft ist es, dass die Komponente D ein anorganisches Oxidationsmittel ist und zwischen 0,01 und 8,4 Gew.% der Komponente D dem Gemisch beigemischt sind. Mit einer Konzentration in diesem Bereich tritt keine Schädigung des Nutzinsektes ein, obwohl der Schädling fast vollständig beseitigt wird.

Zudem ist es vorteilhaft, dass die Komponente C bis zu 100 % reduziert und zumindest eine der Komponenten A oder B oder C oxidiert wird. Der Nachweis, dass das Gemisch bestehend aus den Komponenten A und B im Vorfeld teilweise aufoxidiert wurde, bevor die Komponente C beigemischt wurde, ist möglich.

Als Oxidationsmittel wird in vorteilhafter Weise eine 3-bis 100-prozentige Wasserstoffperoxidlösung als Komponente D dem Stoffgemisch zugemischt. Die Lösung von Wasserstoffperoxid wird erfindungsgemäß dem Gemisch der Komponenten A und B zugegeben, woraufhin der Oxidationsprozess ausgelöst wird.

Alternativ ist erfindungsgemäß vorgesehen, das Gemisch der Komponenten A und B einem anderen Oxidationsprozess unter Einsatz von Luft oder von Sauerstoff zu unterziehen und danach die Komponente C beizumischen. Hierzu wird über eine Lanze die Luft oder das Gas in das Gemisch eingeblasen.

Vorteilhaft ist es, dem Gemisch eine Komponente E bestehend aus mindestens einem aromatischen Alkohol der allgemeinen Formel R4-OH, worin R4 mindestens ein an die Hydroxylgruppe anschließender ringförmiger Arylrest ist, zur Desinfizierung beizumischen. Bevorzugt werden ringförmige Arylreste mit 6 Kohlenstoffatomen. Dabei ist es erfindungsgemäß wesentlich, die Komponente E vor der Zugabe der Komponente D dem Gemisch der Komponenten A und B beizumischen. Dadurch wird erreicht, dass die Komponente E allein oder in Kombination mit einer der anderen Komponenten A oder B durch die Komponente D oxidiert wird. Es sind mindestens zwischen 0,05 und 1,9 Gew.% der zusätzlich beteiligten Komponente E vorzusehen.

Aromatische Alkohole gehören wie die Alkohole zu den Verbindungen mit einfachen funktionellen Gruppen, wodurch die Wirkung der zuvor beschriebenen Stoffe durch das Phenol nicht beeinflusst wird. Das Phenol hat neben einer betäubenden Wirkung zudem ähnliche diffundierende und fettlösende Wirkungen wie der Alkohol und das Keton.

Besonders vorteilhaft ist es, als Komponente E zumindest 2-Biphenylol (2-Hydroxybiphenyl oder 2-Phenylphenol) mit der Summenformel C₆H₆-C₆H₄-OH beizumischen. 2-Biphenylol hat gegenüber anderen aromatischen Alkoholen die vorteilhafte Eigenschaft, das Gemisch zu stabilisieren. Stabilisieren bedeutet, einer Entmischung oder Fraktionstrennung vorzubeugen und chemische Reaktionen unter den einzelnen Stoffen zu unterbinden oder einzudämmen. Ferner hat das 2-Biphenylol gegenüber anderen aromatischen Alkoholen den Vorteil, dass es weniger sauer ist. Ein weiterer Vorteil des 2-Biphenylols gegenüber dem Phenol ist, dass 2-Biphenylol gegenüber Phenol nicht kanzerogen (krebserregend) ist.

Das Gemisch enthält als weitere Komponente F Wasser (H₂O). Für die erfindungsgemäße Wirkung ist es notwendig, dass die Konzentrationen des gesamten Gemischs und der einzelnen Bestandteile für sich ausreichend hoch sind, um den Schädling zu vernichten, aber nicht zu hoch sind, um Schaden an dem zu behandelnden Tier anzurichten. Hierzu ist es vorteilhaft, das Gemisch mit Wasser (H₂O) zu verdünnen. Das Gemisch enthält erfindungsgemäß vor der Zugabe der Komponente D zwischen 30 und 50 Gew.% der zusätzlich beteiligten Komponente F. Es ist anzunehmen, dass das Wasser nicht an der Redox-Reaktion beteiligt ist, jedoch kann es auch nicht ganz ausgeschlossen sein. Es ist allein aufgrund der Stoffeigenschaften von Wasserstoffperoxid ratsam, zuerst Wasser beizumischen.

Es ist weiterhin vorgesehen, das Gemisch auf sonstige Träger von tierischen Schädlingen aufzutragen. Zu den Trägern zählen insbesondere Pflanzen wie bspw. Bäume und Sträucher sowie deren Früchte, Gräser, Blumen, Moose und Textilien wie bspw. Betttücher, Matratzen, Kleidung, Wäsche, die von tierischen Schädlingen befallen werden oder als Nest des Schädlings dienen. Auch bei dieser Anwendung haben die Stoffe A, B, C und D die erfindungsgemäße, durch einen Oxidationsprozess wechselwirkende Eigenschaft, den Schädling zu betäuben und das ZNS zu beschädigen. Die gesundheitliche Unbedenklichkeit des erfindungsgemäßen Gemisches ist auch in diesem Anwendungsbereich von großer Bedeutung, da die behandelten Sachen in unmittelbaren Kontakt mit dem Menschen kommen bzw. als Nahrung dienen. Bspw. im Bereich des Rebschutzes findet das Gemisch zur Bekämpfung des Traubenwicklers in allen drei Generationen, der Heuwurm-, der Sauerwurm- und der Süßwurm-Generation, Anwendung.

Bezüglich einer wirksamen Stoffzusammensetzung ist es vorteilhaft, dass das Gemisch zwischen 50 und 70 Gew.% der Komponente A und zwischen 0,05 und 9,6 Gew.% der Komponente B und zwischen 0,05 und 3,8 Gew.% der Komponente C enthält, wobei die Komponente D zunächst dem Gemisch der Komponenten A, B und ggf. E beigefügt und nachträglich die Komponente C zugemischt wird. Die Konzentrationen der einzelnen Komponenten A bis F sind sehr stark von der Art des zu behandelnden Nutzinsekts und von der Größe des Parasiten abhängig. Bei größeren Tieren und beim Menschen können die Konzentrationen wesentlich erhöht werden.

Das Gemisch findet in vorteilhafter Weise als Medikament zur Behandlung von wirbellosen Tieren und Wirbeltieren Anwendung. Diesbezüglich ist die Verwendung eines vorstehend beschriebenen Gemischs zur Herstellung eines Medikaments für die Behandlung des tierischen Körpers bei der Bekämpfung von Schädlingen von Vorteil. Das Medikament wird in flüssiger Form, in der auch die einzelnen Stoffe A bis F bei Raumtemperatur unter Normaldruck vorliegen, hergestellt.

Die Herstellung zumindest eines Teils des Gemischs als Gel oder in Pulverform ermöglicht es, das für die Applikation notwendige Gemisch erst unmittelbar vor der Applikation bspw. mit Wasser zu versetzen.

Es ist vorteilhaft, das Gemisch zur Herstellung eines Medikaments für die Behandlung von Gliedertieren bei der Bekämpfung von Schädlingen zu verwenden. Schädlinge wie bspw. Milben, Läuse und Nematoden gehören zu der Familie der wirbellosen Tiere, die im Falle von Milben ein Chitinskelett haben.

Eine äußerst vorteilhafte Verwendung findet das Gemisch bei der Herstellung eines Medikaments für die Behandlung von Honigbienen bei der Bekämpfung von Varroamilben (Varroa destructor oder Varroa jacobsoni). Mit dem erfindungsgemäßen Gemisch wurde erreicht, dass der Milbenbefall schon nach einem Applikationszeitraum von 6 bis 8 Wochen um 95 % gegenüber nicht behandelten Referenzvölkern zurückging, ohne dass dabei ein nennenswerter Schaden am aktiven Bienenvolk und an der Brut erkennbar gewesen wäre. Auch wurde gefunden, dass das Gemisch präventiv appliziert werden kann und dadurch ein Milbenbefall weitestgehend ausbleibt.

Bei der Bekämpfung von Milben wird das Gemisch von außen appliziert. Es dringt über die Cuticulla und durch zwangsweise orale Aufnahme, wie eingangs durch den Benetzungsvorgang beschrieben, in die Milbe ein und beschädigt das ZNS sowie den Bewegungsmechanismus durch den sich einstellenden Fettentzug. Die Konzentration der im Gemisch wirksamen Stoffe ist dabei so gering, dass das zu behandelnde Tier wie die Biene keinen Schaden nimmt, da die Biene um ein Vielfaches größer als die Milbe ist.

Untersucht wurde die Auswirkung des Gemischs bei der Behandlung von Honigbienen mit Varroamilbenbefall.

Für die Versuchsanordnung 2004 wurden zwei Bienenvölker (A + B) ausgewählt, die für die Honiggewinnung keine Bedeutung haben, zum einen wegen des angewandten Mittels für anschließende Auswertung auf Rückstände, zum anderen, weil Volk B ein Schwarmvolk darstellte. Die Bienenvolkstärke betrug je ca. 25.000 Tiere. Beide Völker sind in sogenannten Herolds- oder Zeitlerbeuten mit je zwei Beuten à 10 Zargen untergebracht und wurden auf ihren Besatz, ihre Völkerstärke, Brut und ihren allgemeinen Zustand hin untersucht und für gut befunden.

Zu Beginn wurde lediglich Stock A mit dem Gemisch behandelt, Stock B blieb daneben zur Ermittlung von Referenzdaten unbehandelt. Im Zeitraum von 3½ Monaten fanden sieben Behandlungen statt, wobei zwischen zwei Behandlungszyklen eine längere Pause von mehr als 30 Tagen eingelegt wurde. Abschließend fanden zwei Behandlungen mit Perizin^{®} zur Kontrolle der Restmilbenbestände statt.

Zum Einbringen des Gemischs in die Beute wurde ein alkoholbeständiger und säurefester, aus Polyethylen bestehender Druckpumpsprüher, versehen mit einer 30 cm Sprühlanze, verwendet und laut Herstellerempfehlung eingesetzt.

Je Beute (entspricht 10 Zargen) wurden ca. 70 ml Gemisch je Behandlung eingesprüht. Die Beute wurde geöffnet und das Mittel von oben mit der Sprühlanze in einem Abstand von 5 cm in die einzelnen Zwischenräume der Zargen fein eingebracht. Zuerst wurde die untere, direkt danach die obere Beute behandelt.

Bevor die Beute wieder abgedeckt wurde, wurde eine ca. zweiminütige Abluftzeit eingehalten. Der Zeitaufwand je Stock betrug (ohne Lüftung) ein bis zwei Minuten.

Um die Abfallquote an Varroamilben zu ermitteln, wurde der Boden beider Stöcke (A + B) zuvor mit je zwei "Windeln" ganzflächig ausgelegt und mit Vaseline bestrichenem Papier bedeckt. Dieses wurde verwendet, um etwaige noch lebende Varroen am Weglaufen zu hindern. Dieser Vorgang wurde nach jeder Milbenauszählung wiederholt.

Die Völker C bis L stellten Referenzvölker dar und dienten der Ermittlung der Varroadichte am Standort (bei diesen wurde jeweils nur eine Windel eingelegt). Da es sich dabei mehrheitlich um Wandervölker handelte, standen sie nicht alle die ganze Dauer der Behandlung zur Verfügung, spiegelten dennoch ein repräsentatives Bild der allgemeinen Situation wider. Gerade weil viele der Imker mit ihren Völkern wandern, ist trotz Vorsorgemaßnahmen ein Befall mit Varroa stets gegeben.

Bei der Auszählung wurden gefundene Milbenmengen bis 50 Tiere exakt erfasst. Größere Mengen wurden angezählt und anschließend hochgerechnet, da dies bei dieser Größenordnung des Befalls hier keine Rolle für das Testergebnis spielte.

Am 30.08.2004 wurde bei Volk A eine Wachs- und Honigprobe entnommen und auf Rückstände des Gemischs hin untersucht. In beiden Fällen wurden keinerlei Rückstände nachgewiesen.

Der Verlauf wird an dieser Stelle in Stichpunkten aufgeführt.
- 06.06.2004 Erstbehandlung. Einbringen der Windeln in Stock A und B. Überprüfung der Völker auf Besatz, Königin, Brut und Allgemeinzustand.
- 16.06.2004 Kontrolle. Keine Milben. Zweitbehandlung mit Gemisch bei Volk A.
- 27.06.2004 Kontrolle. Keine Milben. Behandlung mit Gemisch bei Volk A.
- 06.07.2004 Kontrolle. Keine Milben. Behandlung mit Gemisch bei Volk A.
- 19.07.2004 Kontrolle. Keine Milben. Keine Behandlung bei Volk A.
- 26.07.2004 Kontrolle. Keine Milben. Zusätzlich Windeln bei Völkern C bis F wegen Erfassung des natürlichen Milbenfalls.
- 02.08.2004 Kontrolle. Auszählung: A + B je 2 Milben. C 18, D 8, E 6, F 6.
- 10.08.2004 Kontrolle. Auszählung: A 0, B 3, C 16, D 14, E 6, G 0. Volk G kam zur Auswertung hinzu. A wurde nochmals mit Gemisch behandelt (zweites Intervall).
- 18.08.2004 Kontrolle. Auszählung: A 1, B 7, C 28, D 20, E 14, G 28, F 0. A wurde nochmals behandelt.
- 23.08.2004 Kontrolle. Auszählung: A 1, B 1, C 40, D 22, E 14, F 6, G 10. Abschlussbehandlung bei Volk A.
- 30.08.2004 Kontrolle. Auszählung: A 1, B 2, C 58, D 50, E 40, F 6, G 8. Völker H bis L werden hinzugezogen. Um zu ermitteln, wie viele Varroamilben in den Völkern verblieben waren, wurden die Völker A + B mit Perizin^{®} behandelt. Bei den Völkern C bis L kam Ameisensäure (60 %) im Verdunstungsverfahren zum Einsatz.
- 06.09.2004 Kontrolle. Auszählung: A 4, B 60, C 1.200, D 600, E 400, F 50, G 400, H 500, I 50, J 200, K 200, L 200. Erneute Behandlung mit Perizin^{®} bei Volk A + B. C bis L mit Ameisensäure behandelt.
- 13.09.2004 Kontrolle. Auszählung: A 6, B 6, C 1.200, D 300, E 300, F 8, G 300, H 600, I 100, J 200, K 140, L 200. C bis L wurde mit Ameisensäure behandelt.
- 20.09.2004 Kontrolle. Auszählung: A 1, B 0, C 160, D 40, E 50, F 10, G 100, H 240, I 38, J 44, K 60, L 100.

Helle Milben, also in diesem Fall junge Nymphenstadien, wurden 2004 bei A nicht gefunden. Bei B waren nur einige wenige auszumachen. Bei C bis L wurden bisweilen nur vereinzelt später, d. h. ab dem 06.09.2004, mehr ausgemacht, was darauf schließen lässt, dass das Gemisch auch durch die Verdeckelung der Waben wirksam ist. Denn erst beim Schlupf der Brut wurde der erhöhte Abfall beobachtet und so die in der Wabe verstorbenen Jungtiere mit ausgeschleppt.

Bei den Völkern C bis L wurden bis zum 30.08.2004 auch teilweise lebende, adulte Milben auf den Windeln ausgezählt.

Diese Versuchsreihe wurde mit einer Gemischzusammensetzung durchgeführt, deren exakte Mengenanteile der einzelnen Komponenten aufgrund von derzeit nicht lesbarem Datenmaterial zum Anmeldezeitpunkt nicht zur Verfügung standen.

Neben Bienen wurden auch Spinnen mit dem Gemisch behandelt. Spinnen werden wie eingangs erläutert von Nematoden (Spulwürmern) befallen, die sich als Parasiten im Verdauungstrakt der Spinne ansiedeln. Gegenüber Bienen muss den Spinnen das Gemisch oral appliziert werden, was durch direkte orale Injizierung erreicht wird. Das Gemisch wird durch den Benetzungsvorgang zwangsweise über den Verdauungstrakt bis zu den Nematoden geführt und dabei nicht zersetzt oder verdaut. Die Nematoden nehmen das Gemisch wiederum zwangsweise primär oral auf und kommen aber auch mit dem Gemisch äußerlich in Berührung. Beides führt in Kombination zu einem Absterben der Nematoden. Die Spinne nimmt hierbei keinen Schaden. Auch bei solchen Applikationen kann das Gemisch präventiv eingesetzt werden.

Die Erfindung bezieht sich ferner darauf, das erfindungsgemäße Gemisch zur Herstellung eines Medikaments für die Behandlung des menschlichen Körpers bei der Bekämpfung von tierischen Schädlingen einzusetzen. Insbesondere wird das Gemisch zur Herstellung eines Medikaments zur Bekämpfung von Schädlingen der Familie der wirbellosen Tiere und bevorzugt von Milben oder von Krätzemilben eingesetzt. Zur Bekämpfung von Schädlingen, die auf der Haut angesiedelt sind, ist es neben der vorstehend beschriebenen Applikationsform vorgesehen, das erfindungsgemäße Gemisch als Zusatzstoff in Seifen, Shampoos oder Körperlotionen beizumischen, um einen direkten Kontakt mit dem Schädling herbeizuführen.

## Patentansprüche

1. Gemisch, insbesondere ein Biozid, enthaltend mindestens die Komponenten A, B und C, wobei
I) die Komponente A aus mindestens einem primären und/oder sekundären Alkohol der allgemeinen Formel R1-OH gebildet ist und R1 ein an die Hydroxylgruppe anschließender verzweigter oder unverzweigter Alkylrest mit 1 bis 12, bevorzugt mit 1 bis 6 Kohlenstoffatomen ist und
II) die Komponente B aus mindestens einer Carbonylverbindung der allgemeinen Formel R2-CO-R2' gebildet ist und R2 oder R2' ein an die Carbonylgruppe anschließender verzweigter oder unverzweigter Aryl- oder Alkylrest oder Wasserstoff ist, wobei R2 und R2' gleich oder verschieden sind und
III) die Komponente C mindestens einen Stoff auf Basis der aromatischen Hydroxyverbindung Benzen der allgemeinen Formel C₆H₆ aus der Familie der etherischen Öle enthält,
herstellbar durch
das Mischen von wenigstens einer oder mehrerer der Komponenten A, B und C mit wenigstens einer weiteren Komponente D und das anschließende Zuführen der übrigen Komponenten zu der Mischung mit der Komponente D, wobei die Komponente D ein Oxidationsmittel ist, das mit wenigstens einer der Komponenten A bis C derart reagiert, dass das Oxidationsmittel reduziert wird.

2. Gemisch nach Anspruch 1,
**dadurch gekennzeichnet,**
**dass** die Komponente C wenigstens Phenol der allgemeinen Formel C₆H₅-OH und/oder ein Phenolether der allgemeinen Formel C₆H₅-O-R enthält.

3. Gemisch nach einem der Ansprüche 1 oder 2,
**dadurch gekennzeichnet,**
**dass** die Komponente D ein anorganisches Oxidationsmittel ist und zwischen 0,01 und 8,4 Gew.% der Komponente D dem Gemisch beigemischt sind.

4. Gemisch nach einem der vorhergehenden Ansprüche,
**dadurch gekennzeichnet,**
**dass** die Komponente D bis zu 100 % reduziert und zumindest eine der Komponenten A oder B oder C oxidiert wird.

5. Gemisch nach einem der vorhergehenden Ansprüche,
**dadurch gekennzeichnet,**
**dass** die Komponente D zumindest teilweise aus einer 3-bis 100-prozentigen Wasserstoffperoxidlösung gebildet ist.

6. Gemisch nach einem der vorhergehenden Ansprüche,
**dadurch gekennzeichnet,**
**dass** das Gemisch vor der Zugabe der Komponente D mindestens zwischen
50 und 70 Gew.% der Komponente A und
0,05 und 9,6 Gew.% der.Komponente B und
0,05 und 3,8 Gew.% der Komponente C enthält.

7. Gemisch nach einem der vorhergehenden Ansprüche, welches eine weitere Komponente E enthält,
**dadurch gekennzeichnet,**
**dass** die Komponente E einen aromatischen Alkohol der allgemeinen Formel R4-OH enthält, worin R4 mindestens ein an die Hydroxylgruppe anschließender ringförmiger Arylrest ist, das Gemisch vor der Zugabe der Komponente D mindestens zwischen 0,05 und 1,9 Gew.% der zusätzlich beteiligten Komponente E enthält und die Komponente E durch die Komponente D oxidierbar ist.

8. Gemisch nach einem der vorhergehenden Ansprüche, welches eine weitere Komponente F enthält,
**dadurch gekennzeichnet,**
**dass** die Komponente F H₂O enthält oder aus H₂O gebildet ist und das Gemisch vor der Zugabe der Komponente D zwischen 30 und 50 Gew.% der zusätzlich beteiligten Komponente F enthält.

9. Gemisch nach einem der vorhergehenden Ansprüche,
**dadurch gekennzeichnet,**
**dass** die Komponente A zumindest 1-Propanol mit der Summenformel CH₃-CH₂-CH₂-OH und/oder 2-Propanol mit der Summenformel CH₃-CH-OH-CH₃ enthält.

10. Gemisch nach einem der vorhergehenden Ansprüche,
**dadurch gekennzeichnet,**
**dass** die Komponente B zumindest Aceton (Propanon) mit der Summenformel CH₃-CO-CH₃ enthält.

11. Gemisch nach einem der vorhergehenden Ansprüche,
**dadurch gekennzeichnet,**
**dass** die Komponente E zumindest 2-Biphenylol (2-Hydroxybiphenyl oder 2-Phenylphenol) mit der Summenformel C₆H₆-C₆H₄-OH enthält.

12. Verwendung eines Gemisches, insbesondere eines Biozids, nach zumindest einem der vorstehenden Ansprüche zur Herstellung eines Medikaments für die Behandlung des tierischen Körpers bei der Bekämpfung von tierischen Schädlingen.

13. Verwendung eines Gemisches nach zumindest einem der vorstehenden Ansprüche 1 bis 12 zur Bekämpfung von tierischen Schädlingen.

14. Verwendung eines Gemisches nach zumindest einem der vorstehenden Ansprüche 1 bis 12 zur Herstellung eines Medikaments für die Behandlung des menschlichen Körpers bei der Bekämpfung von tierischen Schädlingen.

## Claims

1. Mixture, in particular a biocide, comprising the components A, B, and C, wherein
I) component A comprises a primary and/or secondary alcohol of the general formula R1-OH and R1 is a branched or linear alkyl radical with 1 to 12 preferably 1 to 6 carbon atoms connected to the hydroxyl group and
II) component B comprises a carbonyl compound of the general formula R2-CO-R2' and R2 or R2' is a branched or linear aryl or alkyl radical or hydrogen connected to the carbonyl group, R2 an R2' being identical or different and
III) component C comprises a substance on the basis of the aromatic hydroxyl compound benzene of the general formula C₆H₆ from the family of essential oils
which can be produced by way of
mixing at least one or several of the components A, B, and C with one other component D and subsequently adding the remaining components to the mixture containing component D, component D being an oxidising agent which reacts with one of the components A to C in such a way that the oxidising agent is reduced.

2. Mixture according to claim 1, wherein component C comprises phenol of the general formula C₆H₅-OH and/or a phenyl ether of the general formula C₆H₅-O-R.

3. Mixture according to one of the claims 1 or 2 wherein component D is an inorganic oxidising agent and between 0.01 and 8.4 % in weight of component D are added to the mixture.

4. Mixture according to one of the antecedent claims, wherein component D is reduced by up to 100 % and at least one of the components A or B or C is oxidised.

5. Mixture according to one of the antecedent claims, wherein component D is at least partially formed by a 3 to 100 % hydrogen peroxide solution.

6. Mixture according to one of the antecedent claims, wherein the mixture contains at least between
50 and 70 % in weight of component A and
0.05 and 9.6 % in weight of component B and
0.05 and 3.8 % in weight of component C
before component D is added.

7. Mixture according to one of the antecedent claims, which contains a further component E, wherein component E contains a an aromatic alcohol of the general formula R4-OH, R4 being at least one annular aryl radical connected to the hydroxyl group, the mixture contains at least between 0.05 and 1.9 % in weight of the additional component E before component D is added and component E can be oxidised be component D.

8. Mixture according to one of the antecedent claims, which contains a further component F, wherein component F contains H₂O or is made from H₂O and the mixture contains between 30 and 50 % in weight of the additional component F before component D is added.

9. Mixture according to one of the antecedent claims, wherein component A contains at least 1-propanol of the molecular formula CH₃-CH₂-CH₂-OH and/or 2-propanol of the molecular formula CH₃-CH-OH-CH₃.

10. Mixture according to one of the antecedent claims, wherein component B comprises acetone (propanone) of the molecular formula CH₃-CO-CH₃.

11. Mixture according to one of the antecedent claims, wherein component E comprises 2-biphenylol (2-hydroxy biphenyl or 2-phenyl phenol) of the molecular formula C₆H₆-C₆H₄-OH.

12. Application of a mixture, in particular a biocide, according to at least one of the claims set forth above for producing a medicine for the treatment of animal bodies in combating animal pests.

13. Application of a mixture according to at least one of the above claims 1 to 12 for combating animal pests.

14. Application of a mixture according to at least one of the above claims 1 to 12 for producing a medicine for the treatment of the human body in combating animal pests.

## Revendications

1. Composition, en particulier un biocide, contenant au moins les composants A, B et C, étant
I) le composant A au moins un alcool primaire et/ou secondaire de formule générale R1-OH et R1 un radical alkyle ramifié ou linéaire lié à un groupement hydroxyle avec 1 à 12, de préférence avec 1 à 6 atomes de carbone et
II) le composant B au moins un composé carbonyl de formule générale R2-CO-R2' et R2 ou R2' un radical aryle ou alkyle ramifié ou linéaire lié à un groupement carbonyl ou hydrogène, étant R2 ou R2' identiques ou différents et
III) le composant C au moins une substance à base du composé hydroxyle aromatique, le benzène, de formule générale C₆H₆ de la famille des huiles essentielles,
qui peut être produit par
le mélange d'au moins un ou plusieurs des composants A, B et C avec au moins un autre composant D et en ajoutant les composants restants au mélange avec le composant D, étant le composant D un oxidant qui réagit avec au moins un des composants A à C de manière que l'oxidant se réduit.

2. Composition selon la revendication 1,
**caracterisée par le fait**
**que** le composant C contient au moins phénol de formule générale C₆H₅-OH et/ou un éther de phénol de formule générale C₆H₅-O-R.

3. Composition selon une des revendications 1 ou 2,
**caracterisée par le fait**
**que** le composant D est un oxidant inorganique et que entre 0,01 et 8,4 % en poids du composant D est mélangé à la composition.

4. Composition selon une des revendications précédentes,
**caracterisée par le fait**
**que** le composant C se réduit jusqu'à 100 % et au moins un des composants A ou B ou C s'oxyde.

5. Composition selon une des revendications précédentes,
**caracterisée par le fait**
**que** le composant D est formé au moins en partie d'une solution de 3 à 100 % de péroxyde d'hydrogène.

6. Composition selon une des revendications précédentes,
**caracterisée par le fait**
**qu'**avant d'y ajouter le composant D, la composition contient au moins entre
50 et 70 % en poids du composant A et
0,05 et 9,6 % en poids du composant B et
0,05 et 3,8 % en poids du composant C.

7. Composition selon une des revendications précédentes, qui contient un autre composant E,
**caracterisée par le fait**
**que** le composant E contient un alcool aromatique de formule générale R4-OH, le R4 étant au moins un radical aryle cyclique lié au groupement hydroxyle, la composition contenant avant d'y ajouter le composant D au moins entre 0,05 et 1,9 % en poids de l'additionnel composant E et le composant E étant oxydable par le composant D.

8. Composition selon une des revendications précédentes, qui contient un autre composant F,
**caracterisée par le fait**
**que** le composant F contient H₂O ou est formé par H₂O et que la composition avant d'y ajouter le composant D contient entre 30 et 50 % en poids de l'additionnel composant F.

9. Composition selon une des revendications précédentes,
**caracterisée par le fait**
**que** le composant A contient au moins propan-1-ol de formule CH₃-CH₂-CH₂-OH et/ou propan-2-ol de formule CH₃-CH-OH-CH₃.

10. Composition selon une des revendications précédentes,
**caracterisée par le fait**
**que** le composant B contient au moins acétone (propane) de formule CH₃-CO-CH₃.

11. Composition selon une des revendications précédentes,
**caracterisée par le fait**
**que** le composant E contient au moins 2-biphénylol (2-hydroxybiphényle ou 2-phényl-phénol) de formule C₆H₆-C₆H₄-OH.

12. Utilisation d'une composition, en particulier d'un biocide, selon au moins une des revendications précédentes pour la fabrication d'un médicament destiné au traitement du corps animal dans la lutte contre les animaux nuisibles.

13. Utilisation d'une composition selon au moins une des revendications précédentes 1 à 12 destinée à la lutte contre les animaux nuisibles.

14. Utilisation d'une composition selon au moins une des revendications précédentes 1 à 12 pour la fabrication d'un médicament destiné au traitement du corps humain dans la lutte contre les animaux nuisibles.
